# EUROPEAN PATENT SPECIFICATION

(11) **EP 2 152 185 B1**
(45) Date of publication and mention of the grant of the patent: **23.05.2012**
(21) Application number: 08756599.0
(22) Date of filing: 02.06.2008
(51) Int. Cl.: A61B 19/00, A61B 17/34, A61B 8/08, A61N 1/34

(54) **CONTINUOUS NERVE BLOCK ASSEMBLY**
ANORDNUNG ZUR KONTINUIERLICHEN NERVENBLOCKAGE
ENSEMBLE POUR BLOC NERVEUX CONTINU

(30) Priority: 08.06.2007 US 811433
(43) Date of publication of application: 17.02.2010
(73) Proprietor: Cook Medical Technologies LLC, Bloomington, IN 47404 (US)
(72) Inventor: ARNDT, George, A., Madison, WI 53719 (US); BOSEL, Christopher, D., Bloomington, IN 47404 (US)
(74) Representative: Jehan, Robert
(86) International application number: PCT/US2008/065485
(87) International publication number: WO 2008/154200

(56) References cited:
- WO-A-01/21081
- WO-A-89/11250
- WO-A-2005/020905
- WO-A-2007/070374
- US-A- 5 201 314
- US-A- 6 018 676
- US-B1- 6 298 256
- DATABASE MEDLINE [Online] US NATIONAL LIBRARY OF MEDICINE (NLM), BETHESDA, MD, US; January 2006 (2006-01), BELL GRAHAM T ET AL: "Caudal catheters and ultrasound." XP002493126 Database accession no. NLM16409545 & PAEDIATRIC ANAESTHESIA JAN 2006, vol. 16, no. 1, January 2006 (2006-01), pages 98-99 ; auth, ISSN: 1155-5645
- SCHAFHALTER-ZOPPOTH I ET AL: "Ultrasound visibility of needles used for regional nerve block: An in vitro study" REGIONAL ANESTHESIA AND PAIN MEDICINE, CHURCHILL LIVINGSTONE, SECAUCUS, NJ, US, vol. 29, no. 5, 1 September 2004 (2004-09-01), pages 480-488, XP004563646 ISSN: 1098-7339
- NICHOLS ET AL: "Changes in Ultrasonographic Echogenicity and Visibility of Needles with Changes in Angles of Insonation" JOURNAL OF VASCULAR AND INTERVENTIONAL RADIOLOGY, VA, vol. 14, no. 12, 1 December 2003 (2003-12-01), pages 1553-1557, XP022010107 ISSN: 1051-0443

## Description

### BACKGROUND

1. Technical Field. The present invention relates generally to an assembly for use in blocking a peripheral nerve of a patient to produce regional anesthesia. More particularly, the invention relates to an echogenic continuous nerve block assembly suitable for such use. The invention further relates to a method for continuously blocking a nerve.

2. Background Information. It is a well known medical practice to produce regional anesthesia in a patient by depositing a local anesthetic along the path of one or more peripheral nerves. The success of the technique is largely dependent upon the ability of the clinician to deposit the anesthetic in close proximity to the nerve. Local anesthetics comprise a class of drugs which reversibly interact with a nerve in a manner such that the propagation of signals along the nerve fiber is significantly reduced, or stopped altogether, for a limited period of time. When such drugs are deposited along large nerve trunks, such as the femoral nerve in the groin or the nerve trunk of the brachial plexus in the axilla or neck, the effect is to make the targeted body structure, such as a body limb, insensate or "numb."

When the local anesthetic is intended to be injected into the groin, neck or axilla, the relevant nerve or nerves must, of course, be located before the injection is given. A general understanding of surface anatomy allows the general area of the nerve to initially be located. Historically the nerve was located by eliciting a paraesthesia, or pain, resulting from the needle coming into contact with the nerve fiber. This is very similar to the sensation experienced by hitting the "crazy bone", where the ulnar nerve is stimulated by pressure being placed on it between the skin and the bone. When this process is done with a needle, the risk of damaging a nerve fiber is high, with the possible result of permanent nerve injury. This technique has been largely abandoned due to the high possibility of such permanent injury.

The use of nerve blocks to accomplish such anesthesia has now progressed to the point where a stimulating needle may be utilized to locate a nerve, without making direct contact with the nerve. Nerve block systems are provided with certain features to minimize damage to the nerve. A first feature is to utilize a beveled needle end cut at an angle. Typically, such needles are provided to have tip angles of about 15°, 30° or 45°. A more blunt needle allows the user to feel the tissue during insertion, and is less likely to cause damage to the nerve. A second feature is to provide the needle with a coaxial design, consisting of a needle shaft covered with a plastic coating, such as PTFE. The needle shaft is connected to an electrode, and the needle electrode system and a grounding skin electrode are connected to a commercially available nerve stimulation box.

An electrical circuit is formed when the needle is placed in the patient's tissue and the grounding electrode is connected to the patient's skin, e.g., with a conventional EKG electrode. The nerve stimulation resulting from this circuit is capable of delivering adjustable pulses of electrical energy through the needle. When the needle tip is in close proximity to the nerve, the motor nerve fibers are stimulated to cause muscles innervated by the nerve to twitch by electrical stimulation resulting from the electrical current flow in the electrical circuit. This mechanism is similar to that observed in a high school biology experiment, when the leg of a freshly dead frog is made to twitch by direct electrical stimulation of the nerve, thereby innervating the leg. In this nerve stimulation technique, the clinicians are, in effect, attempting to localize the nerve without actually puncturing the nerve tissue. The technique is intended to allow the needle to come close to the nerve, without actually contacting the nerve fibers in a manner that might cause damage to the nerve.

Needle insertion by the aforementioned technique is based upon clinical judgment, and therefore, is not precise. The amount of electric current necessary to make the correct muscle twitch for the nerve to be blocked is determined by the proximity of the needle to the nerve. Generally, only a small amount of current is required, since resistance is typically minimal as the needle approaches the nerve. In clinical practice, this is typically performed at 1 to 4 Hz stimulation frequency, with an optimal current of 0.5 milliamps or less to bring the needle in close enough proximity to the nerve for drug injection. The actual voltage required is proprietary, and is a property of the particular peripheral nerve stimulator utilized in the technique. It is set at a value to produce a motor response without pain. A limitation of this technique is that it is a blind technique that is carried out based on a general understanding of the surface anatomy of the particular nerve to be blocked, and without a precise location of the nerve under the skin.

Ultrasound energy comprises high frequency sound waves generated in the 2 to 15 MHz range. In common medical practice, a range of 5 to 12 MHz is employed for most applications, as this range provides optimal tissue resolution and penetration. The sound waves are commonly generated using a piezoelectric crystal. Piezoelectric crystals produce ultrasound energy when electrically stimulated, and also respond to reflected ultrasound energy. The ultrasound energy is pulsed and time locked. Ultrasound energy is typically reflected, and this reflected ultrasound energy is capable of amplification. Measuring reflected amplified energy enables the clinician to determine a range or distance to a tissue interface. Medical ultrasound techniques, such as 2D medical ultrasound, typically employ a piezoelectric effect reflective head, a computer, an electronic component, and a monitor to display the anatomy generated by the ultrasound integration of the tissue being examined.

A 2D ultrasound technique typically uses an ultrasound head with a set of piezoelectric crystals in alignment, which crystals can be electronically switched on or off to respond to reflected ultrasound energy. The time delay between ultrasound emission and reflection can be used to construct a 2D picture of the tissue in alignment in the ultrasound plane generated. When the piezoelectric crystals are switched on and off electronically, a planar picture of the anatomy is created and displayed on the 2D ultrasound monitor. The 2D ultrasound machine allows tissue and anatomy to be visualized in both the axial and lateral direction. By controlling the switching order and timing of the individual piezoelectric crystals in the ultrasound head, the tissue can be scanned in a temporal fashion, thus creating a real time display of the tissue, and thus motion.

Ultrasound techniques, such as 2D ultrasound, are widely used in modern medicine. Such techniques are currently used for peripheral nerve blockage by allowing the clinician to view the nerve to be blocked in real time. In using a 2D ultrasound machine to block a nerve, the clinician is able to see below the skin, and thereby view the location of the nerve or nerves to be blocked. This renders greater precision in the procedure, and allows the clinician to advance the needle to the desired position relative to the nerve. A local anesthetic can then be deposited near the nerve to be blocked.

Conventional nerve stimulating block needles are typically of coaxial design. These needles have an inner needle portion made of metallic material, typically surgical grade steel. A plastic matrix covers most of the length of the needle, and extends generally from the proximal end of the needle nearly to the bare metal needle tip. This type of needle construction ensures maximal current density, as the current can only exit at the unencased metal needle tip. The plastic covering of the needle insulates the remaining portion of the needle from the remaining patient tissue, ensuring that electrical current primarily exits at the needle tip. The needle tip, when in close proximity to the nerve, localizes the nerve with electrical stimulation while minimizing nerve damage.

One major shortcoming of the 2D ultrasound technique is that the needle is often not easily visible in the plane of the 2D ultrasound beam. Maximum reflection of ultrasound energy occurs when the needle is at a 90° angle to the direction of the ultrasound waves in the 2D ultrasound plane. The signal degrades as this angle is reduced, to a point at which the needle becomes invisible in the 2D ultrasound plane. As a result, it is often ergonomically difficult to align the needle with or under the ultrasound head, define the tissue anatomy, and advance the needle in a 3D structure, while keeping the needle in view on the narrow 2D ultrasound plane.

When regional anesthesia is produced in a patient by injecting the local anesthetic along the path of a peripheral nerve utilizing a needle, the anesthesia is generally effective to make the target nerve insensate for a relatively short period of time. This time period is typically a matter of hours, the exact period depending upon the particular anesthetic used, and the specific dosage of the anesthetic injected into the patient. In some surgical procedures, however, it is desired to provide continuous introduction of anesthetic, such that the period of insensitivity can be extended beyond that which is safely available from a single injection, such as for a period of several days. In such cases, multiple, spaced doses may be injected into the patient to provide continuous analgesia. The use of multiple injections may be traumatic to the patient, and also requires the technician to locate multiple suitable injection sites on the skin of the patient. As an alternative, a catheter may be inserted through the barrel of the needle, which catheter is in fluid communication with a source of anesthetic. An infusion pump can be used to continuously pump anesthetic through the catheter to the target site for the designated period of time. However, when techniques such as 2D ultrasound are utilized, the distal tip of the catheter may not be visible under ultrasound. As a result, it can be difficult to determine whether the distal tip of the catheter has been optimally placed relative to the nerve.

It would be desirable to provide a nerve block assembly that utilizes an injection needle for an anesthetic, which needle is capable of simultaneous nerve stimulation and ultrasound visualization. It would also be desirable to include a catheter within the assembly capable of transmitting an anesthetic to a target site for continuous nerve block. WO2007107374, an earlier application by Cook Critical Care Incorporated, describes the use of hyperechoic stimulating block needle which provides a mechanism for passing a catheter through the needle using 2D ultrasound, and for removing the needle leaving the catheter In place for continuous administration of drugs. WO2007/07374 discloses that the catheter may have an echogenic material incorporated in the catheter matrix, or at the tip of the catheter matrix, to enable visualization and advancement of the catheter through the needle, and correct anatomical position using ultrasound.

### BRIEF SUMMARY

The present invention addresses the limitations of the prior art. An assembly is provided for continuous blockage of a nerve of a patient. The assembly includes a needle comprising a hollow needle conduit having a shaft portion and a tip portion, a generally non-conductive layer disposed along a length of the conduit shaft portion, and an echogenic surface extending along at least a portion of the needle. A catheter is sized to be received in the shaft portion of the needle. The catheter shaft has a bore extending at least partially therethrough for transmittal of an anesthetic to a vicinity of the nerve. The catheter has an echogenic capability, such as an echogenic surface disposed along a portion of its length. The needle is capable of providing electrical stimulation to the nerve, and the respective needle and catheter echogenic surfaces are arranged for enhancing a reflection of ultrasound waves generated during ultrasound visualization.

The invention is defined by claim 1 and comprises an echogenic catheter suitable for ultrasonic visualization during a medical procedure. The catheter comprises a catheter shaft having a proximal end, a distal end, and a bore extending at least partially through the shaft. The catheter shaft is formed from a generally flexible polymeric composition, such as a urethane or a nylon composition. A coiled ribbon is wrapped around at least a distal portion of the catheter shaft. The coiled ribbon, preferably formed from a metal or a metal alloy, includes deformations shaped and positioned along a surface of the ribbon to reflect ultrasound waves, so that the ribbon is visible under ultrasonic visualization.

### BRIEF DESCRIPTION OF THE DRAWINGS

Fig. 1 illustrates a perspective view of a hyperechoic stimulating block needle according to one embodiment of the present invention;

Fig. 2 illustrates an enlarged view of an embodiment of a tip member for a hyperechoic stimulating block needle;

Fig. 3 is a diagram illustrating a hyperechoic stimulating block needle inserted into a patient approaching a peripheral nerve, using a 2D ultrasound machine and peripheral nerve stimulation;

Figs. 4 and 4A are schematic diagrams illustrating the interaction between ultrasound waves and a coaxial electrical stimulating peripheral nerve block needle of the prior art;

Fig. 5 is a schematic diagram illustrating the interaction between ultrasound waves and a hyperechoic nerve block needle according to an embodiment of the present invention;

Fig. 6 illustrates a catheter having an echogenic surface suitable for use with the inventive needle;

Fig. 7 illustrates a continuous nerve block assembly according to an embodiment of the present invention, including a needle and an echogenic catheter;

Fig. 8 illustrates an alternative embodiment of a catheter having an echogenic surface; and

Fig. 9 illustrates the deflection of the catheter of Fig. 8 upon contact with tissue.

### DETAILED DESCRIPTION OF THE DRAWINGS AND THE PRESENTLY PREFERRED EMBODIMENTS

For the purposes of promoting an understanding of the principles of the invention, reference will now be made to the embodiments illustrated in the drawings, and specific language will be used to describe the same. It should nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

In the following discussion, the terms "proximal" and "distal" will be used to describe the opposing axial ends of the needle portion of the inventive assembly, as well as the axial ends of various components of the assembly. The term "proximal" is used in its conventional sense to refer to the end of the needle (or component) that is closest to the operator during use of the needle. The term "distal" is used in its conventional sense to refer to the end of the needle (or component) that is initially inserted into the patient, or that is closest to the patient during use.

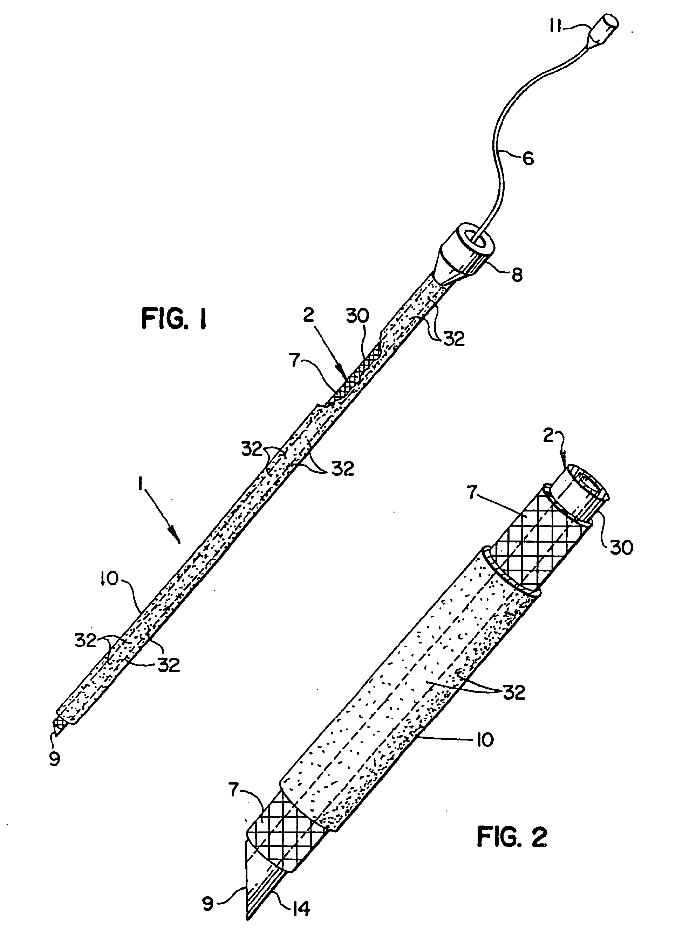

Fig. 1 illustrates a hyperechoic stimulating block needle 1. Among other uses, needle 1 may be utilized in a process for blocking a peripheral nerve that combines nerve stimulation and 2D ultrasound visualization techniques. Needle 1 is provided with an irregular surface that enhances the reflection of ultrasound waves toward an emitter receiver array of a 2D ultrasound machine, thereby allowing enhanced needle visualization with the ultrasound machine. This allows the needle to be inserted into the patient's tissue and advanced toward a peripheral nerve with greater precision than may be achieved with either ultrasound or peripheral nerve stimulation alone. The technique may be utilized to deposit drugs near a nerve, e.g., to produce local or regional anesthesia at a targeted area in the body of the patient.

In the embodiment shown in Fig. 1, needle 1 includes a hollow elongated conduit 2. Conduits for use in stimulating needles are well known in the art, and conduit 2 may have a composition typically utilized for such purpose. Preferably, conduit 2 comprises an elongated shaft portion 30 formed of an electrically conductive metal or metal alloy, such as surgical grade steel, and an electrically conductive distal tip portion 9. Distal tip portion 9 may be formed of the same metal or alloy used for forming shaft 30, or from a different conductive material.

Tip 9 has sufficient sharpness to enable the needle to puncture the patient's skin, and advance through tissue. Preferably, tip 9 is an arcuate or beveled tip, and more preferably, is a short bevel tip. Although the short bevel tip is not restricted to a particular bevel angle, in a preferred embodiment, the bevel angle is about 45°. Those skilled in the art will appreciate that beveled tips at angles other than 45° (such as less than 45°) may be preferred in certain circumstances, and such tips are also within the scope of the invention. Typically, short bevel tips require more force during insertion than long bevels. However, this additional exertion of force enables the clinician to better "feel" the texture of the tissue as the tip is advanced, thereby helping to identify the tip location. Those skilled in the art are very familiar with various needle tips, and are suitably equipped to select a satisfactory tip for a particular application in view of the teachings of the present invention.

An electrically non-conducting insulating layer 7 covers at least a portion of conduit 2. Preferably, insulating layer 7 covers substantially the entire length of conduit shaft portion 30, but does not cover tip portion 9. Providing the insulating covering on shaft portion 30 insulates the shaft from the remaining patient tissue, and ensures that maximal electrical current exits at tip portion 9. Preferably, the covering material is a plastic, such as PTFE. Although insulating layer 7 need not necessarily extend along the entire length of shaft 30, this arrangement is preferred so that maximum current density is provided at the tip.

In the example shown, at least a portion of the electrically non-conducting layer 7 is covered by an echogenic encasing sheath 10. Sheath 10 may comprise a conventional jacket or tube, or may alternatively comprise a coating layer that covers all or part of layer 7. The sheath is preferably formed of a metal or metal alloy, and is provided with an irregular surface. In the example shown, the irregular surface comprises a plurality of deformations 32 distributed along the exterior surface of sheath 10. Deformations 32 are imperfections that are formed along the sheath surface in a manner that enhances the ability of the needle to scatter and/or reflect ultrasound energy back to the ultrasound head, thereby improving the echogenic capacity of the needle. The deformations may be formed along the length of the sheath by well-known processes, such as sandblasting, physical deformation, micro hammering, dimpling, etc. Those skilled in the art will appreciate that there are many other ways of forming surface deformations of a type that will result in the scatter and/or reflectance of ultrasound waves that may be substituted for the techniques described above. Deformations 32 do not adversely affect the mechanical properties of the needle with respect in the ability of the needle to pass through tissue.

The presence of the imperfections, such as deformations 32, causes ultrasound waves that contact the deformations to travel in multiple directions and in a more random fashion than with conventional needles. The increase in scatter and/or reflection of the ultrasound waves enhances the temporal visualization of the hyperechoic needle path and tip during 2D ultrasound examination. This action significantly improves the axial, lateral and temporal resolution of the stimulating needle under 2D ultrasound. In practice, the deformations enhance the visibility of the needle under 2D ultrasound, regardless of the needle orientation to the ultrasound head. The needle will appear on the 2D ultrasound monitor in real time, and the needle position, needle path tip, and interaction with the tissue will be visible in real time. As a result, with a visible echogenic needle, the needle may be safely advanced toward the nerve using the 2D ultrasound monitor.

Although the echogenic sheath has been described herein as a jacket or a coating applied over the insulating layer of the needle, this arrangement is not required. Alternatively, the echogenic surface may comprise the deformation of a surface, such as a cannula or the shaft of the needle, that is positioned under the insulating layer. In this event, the ultrasound beam passes through the insulating layer, and reflects off the cannula or shaft surface back to the ultrasound head. As a still further alternative, the echogenic surface may result from the combined effect of deformation over and under the insulating layer.

The echogenic layer need not be continuous along the length of the needle. Rather, the echogenic layer may be discontinuous along the needle axis, and the length of the needle may include discrete lengths having, and not having, irregularities or deformities. This arrangement provides additional contrast along the needle surface, thereby allowing the clinician to delineate position, path, and length of the needle using 2D ultrasound. Similarly, the echogenic layer need not be structured to provide only a single type of echogenic signal. Rather, the layer may be structured with more than one type of imperfection or deformity, to provide different types of echogenic signals along the needle axis, thereby providing additional contrast and/or visibility along the needle surface.

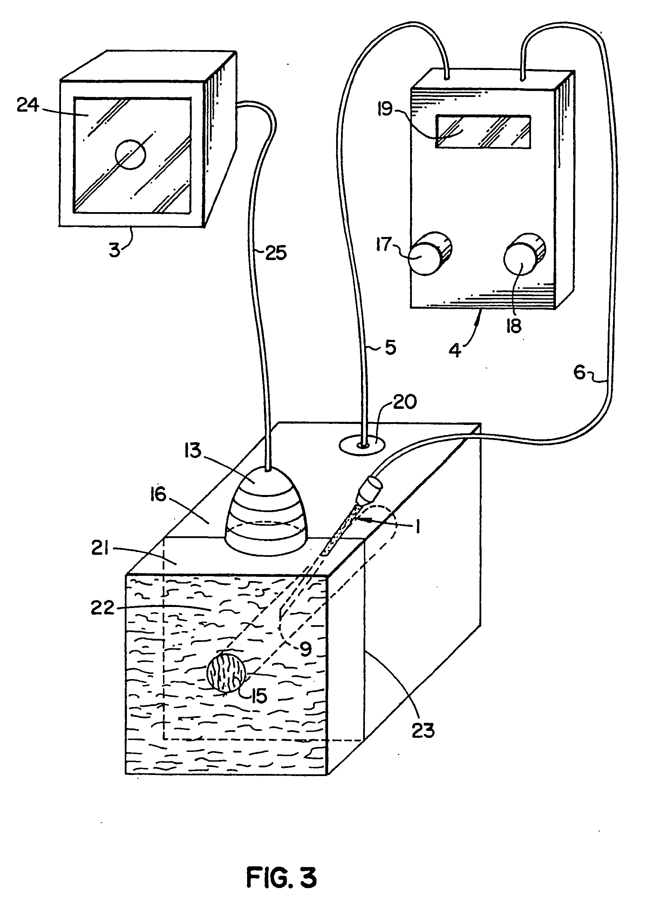

In the example shown, a generally tubular metal or plastic hub 8 is engaged with the proximal end of shaft 30. When present, hub 8 is sized and shaped for attachment to a syringe, tube, or other medical device in well known fashion. Echogenic encasing sheath 10 is preferably electrically isolated from the tubular hub 8 and hollow metal conduit 2. To utilize needle 1 in a circuit, an electrode 6 may be electrically connected at one end to the metal conduit 2 or hub 8, and at the other end to a conventional peripheral nerve stimulator 4 (Fig. 3). The proximal end of electrode 6 may terminate at a mechanical connector 11 of a type that is suitable for connection to an outlet of the peripheral nerve stimulator 4.

Fig. 2 is an enlarged view of the distal end or tip 9 of the hyperechoic stimulating block needle 1 of Fig. 1. The needle tip 9 is bevel cut at an angle of approximately 45°, or in other words, at a less acute angle than a standard needle. The unencased echogenic needle tip portion 14 may also be rendered echogenic by deforming the surface extending from the needle end 9 to the insulating coating 7. As a result, the needle tip can be distinguished from the remaining hyperechoic stimulating block needle 1 as viewed by 2D ultrasound.

Fig. 3 illustrates a system for peripheral nerve block. The system includes a hyperechoic stimulating block needle 1, a medical imaging mechanism 3, and a peripheral nerve stimulator 4. Preferably, the imaging mechanism comprises an ultrasound machine, and more preferably, a 2D ultrasound machine. Those skilled in the art will recognize that other medical imaging mechanisms capable of receiving an array of detectable beams may be substituted for the 2D ultrasound mechanism described in the preferred embodiment herein. Peripheral nerve stimulation devices are known in the art, and a skilled artisan can readily select an appropriate device in view of the teachings herein.

A peripheral nerve 15 of the patient is depicted in Fig. 3 in a block of tissue 16. The remaining portions of the patient's anatomy are not relevant to gaining an understanding of the system, and are therefore not shown in the figure. In the example shown, peripheral nerve stimulator 4 has two controls, namely a frequency control knob 17, and an amperage or current control knob 18. Also, in the example shown, peripheral nerve stimulator 4 is provided with an optional digital readout 19 for displaying the current when a circuit is formed.

A circuit is formed by attaching a grounding electrode 5 that extends from the peripheral nerve stimulator 4 to a conventional electrode 20 of the type that is placed on the patient's skin 21, and a needle electrode 6 that extends from the hyperechoic stimulating block needle 1 to the peripheral nerve stimulator 4. The hyperechoic stimulating block needle 1 is inserted into patient tissue 22. A pathway is thereby formed for electrons to flow from peripheral nerve stimulator 4 through the needle electrode 6 to the hyperechoic stimulating block needle 1, and through the shaft 30 (which is electrically insulated from the patient) and the tip 9 of the needle. The electrons pass through the patient's tissue 22 and exit the patient through skin electrode 20, returning to the peripheral nerve stimulator 4 via grounding electrode 5. Peripheral nerve 15 is located by activating the peripheral nerve stimulator 4 to form the circuit. Stimulator frequency knob 17 is adjusted to emit an electrical pulse, most commonly with a range of 1 to 4 Hz. Stimulator amperage control knob 18 is adjusted to elicit a motor response when the needle is advanced in the region of the peripheral nerve 15. The amperage is commonly set at about 2 milliamps to search for the general nerve location.

The needle is advanced toward the peripheral nerve 15 using general knowledge of surface anatomy, and with the guidance of the 2D ultrasound machine 3. The 2D ultrasound machine 3, shown schematically in Fig. 3, typically comprises a monitor 24, a computer (not shown), a cable or head cord 25 and an ultrasonic head 13. Ultrasonic head 13 typically includes a series of piezoelectric effect crystals in alignment. The ultrasound head 13 is capable of sending out a series of ultrasound beams, and to receive reflected energy. The reflected energy is amplified, processed, and integrated in 2D ultrasound machine 3, thereby rendering a 2D planar image of the tissue below the head.

The nerve 15 is rendered visible in a lateral and axial fashion in the plane of the 2D ultrasound machine 23, and displaced as a planar 2D image on the monitor 24 of the 2D ultrasound machine 3. The signal from the ultrasound machine head 13 is received by the 2D ultrasound machine 3 via the 2D ultrasound head cord 25. The peripheral nerve stimulator 4 emits a square wave DC current at a predetermined voltage (e.g., typically about several hundred volts) that is determined according to the characteristics of the peripheral nerve stimulator. As the hyperechoic stimulating block needle 1 is advanced toward the peripheral nerve 15, the motor response is elicited by stimulation of the motor nerve fibers by current flowing through the nerve. The correct nerve to be blocked can be determined by a general understanding of the anatomy of the nervous system, and in particular, by recognizing which nerve will cause a specific part of the body to move as a result of the electrical stimulation.

The motor response is different for each nerve to be blocked. As the needle is advanced toward the target nerve, the motor response becomes more intense, since less tissue is present between the needle tip 9 and peripheral nerve 15, thereby reducing the resistance to current flow. The current is decreased as the needle approaches the nerve, as less current is required to elicit a motor response. Clinically when the current is at 0.5 milliamps or less, the needle tip 9 is in close enough proximity to the nerve that a local anesthetic can be injected, thereby achieving the desired clinical response of making the area innervated by the nerve 15 insensitive or numb.

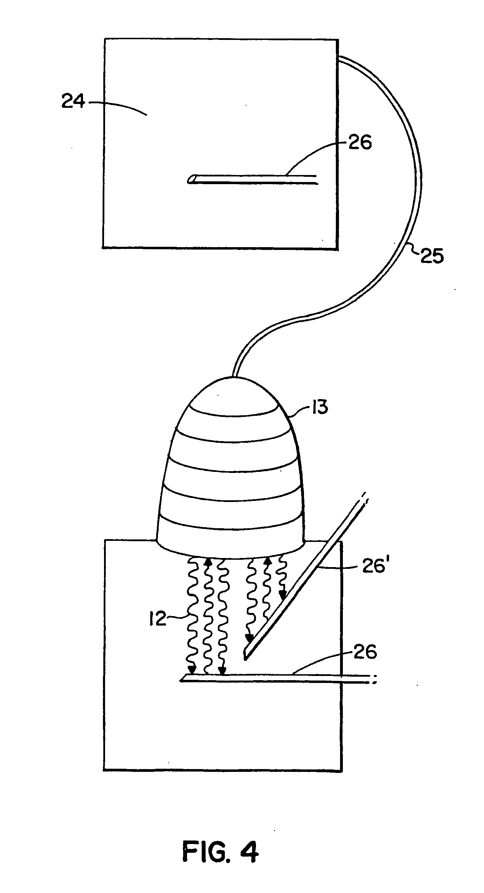

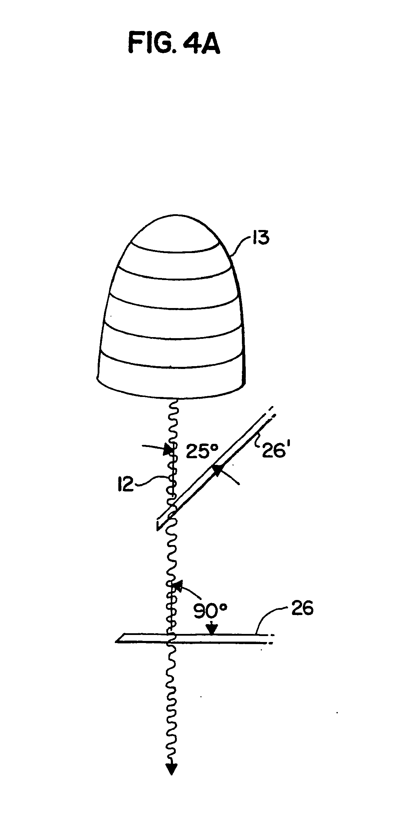

Fig. 4 depicts a display on a 2D ultrasound monitor of a conventional coaxial stimulating block needle 26 in the 2D ultrasound plane 23 in a tissue block. Fig. 4A depicts a display substantially similar to that of Fig. 4, but indicating reference points to various angles cited herein. The coaxial stimulating block needle 26 is seen optimally on monitor 24 when it is oriented at a 90° angle to the ultrasound beam. However, the ability to resolve the coaxial needle image on the 2D ultrasound monitor 24 degrades as the needle moves from the 90° orientation to a lesser orientation, at which point it becomes invisible. Fig. 4A provides a frame of reference for the angles specified. Note in the figure that angled needle 26' is invisible on ultrasound monitor 24. In the example shown, the orientation of the needle 26 has been rotated 65° from the 90° orientation to an angle of 25°. This phenomenon is caused by specular reflectance, as the needle shaft will only reflect ultrasound waves 12 away from the ultrasound head 13. This reflectance is generally similar to the way that light is reflected from a mirror. The specular reflectance of the coaxial block needle 26 makes needle visualization with 2D ultrasound difficult. Commonly, the needle is invisible as it is advanced, greatly decreasing the utility of 2D ultrasound by allowing only peripheral nerve stimulation to resolve needle tip position, resulting from decreasing amperage requirement to elicit a motor response.

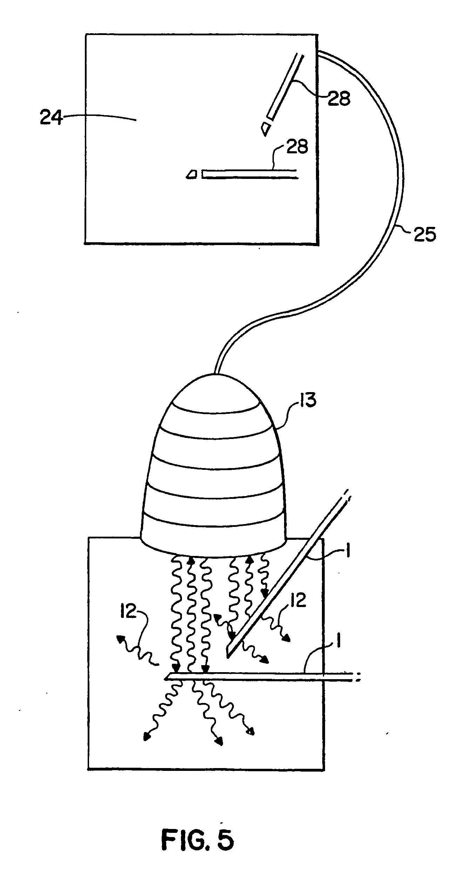

Fig. 5 depicts a display on a 2D ultrasound monitor 24 of a hyperechoic stimulating block needle 1 in a tissue block. The hyperechoic stimulating block needle 1 has an echogenic layer or sheath 10 as described hereinabove, which echogenic layer or sheath is structured to maximally reflect ultrasound waves 12 back to the ultrasound head 13. This renders the hyperechoic stimulating block needle visible 1 as it moves from a 90° orientation (e.g., perpendicular) to the direction of the ultrasound waves to an orientation that approaches an alignment with the ultrasound waves. The hyperechoic stimulating block needle image 28 is thus visible in the 2D ultrasound plane 23 with greater resolution than may be achieved with the coaxial needle 26, both at the 90° angle, and at lesser angles. In Fig. 5, needle image 28 is visible after the needle has rotated 65° from the perpendicular orientation described.

Echogenic layer 10 enables the hyperechoic stimulating needle 1 to be seen as it is advanced in the 2D ultrasound plane 23, rendering both the needle path and needle tip 9 visible as the needle approaches the peripheral nerve 15. This reflection is different than specular reflectance, since it results from the scattering of the ultrasound waves 12 by the echogenic layer 10. In this case, wave scattering occurs toward the 2D ultrasound head 13 to make the hyperechoic stimulating block needle image 28 visible at various angles in the 2D ultrasound plane 23.

The ability to see the needle path, needle tip 9, and the peripheral nerve 15 to be blocked, in combination with peripheral nerve stimulation, allows the peripheral nerve 15 to be approached with more precision. The position of the needle can be resolved anatomically using 2D ultrasound by simultaneous visibility of the needle path, needle tip 9 and peripheral nerve 15, and can be resolved physiologically by using peripheral nerve stimulation to elicit a motor response by minimizing the current. This allows the needle tip 9 to be directed to a closer proximity to the peripheral nerve 15 when compared to the use of 2D ultrasound, peripheral nerve stimulation and a coaxial peripheral stimulating nerve block needle, as shown in Fig. 4. As a result, drugs can be deposited more precisely than by using either ultrasonic visualization or peripheral nerve stimulation separately.

When regional anesthesia is produced in a patient by injecting an anesthetic along the path of a peripheral nerve utilizing a needle, such as hyperechoic block needle 1, the anesthesia is generally only effective for making the target nerve insensate for a relatively short period of time. This time period is typically a matter of hours, the exact period depending upon the particular anesthetic used, and the specific dosage of the anesthetic injected into the patient. In many surgical or pain management procedures, however, it is desired to provide continuous introduction of anesthetic such that the insensate, or numbing, sensation is effective for several days, rather than for a small number of hours. In these procedures, the needle is initially used to locate the nerve, and access the neurovascular sheath. A small amount of anesthetic is injected within the neurovascular sheath. A local expansion within the neurovascular sheath is created, which expansion makes more room for catheter placement. A catheter is then inserted through the bore of the needle into the neurovascular sheath. The needle is reversed over the catheter, while the catheter is held in position. Following removal of the needle, the needle may be discarded. The catheter is tunneled under the skin, and/or a fixation device may be used to hold the catheter in place. A conventional quick-connect hub, such as a Tuohy-Borst connection or the like, is connected to the proximal end of the catheter, and the hub is attached to an anesthetic delivery system.

When techniques such as 2D ultrasound are utilized as described herein, the distal tip of the catheter may not be visible under ultrasound, or it may only be visible at high resolution. As a result, it can be difficult to determine whether the distal tip of the catheter has been properly placed relative to the nerve such that optimal benefit may be achieved from the anesthetic. Thus, another feature of the invention involves the use of a catheter for continuous administration of anesthetic, and more particularly, a catheter having an echogenic capability such that the catheter is visible under ultrasound.

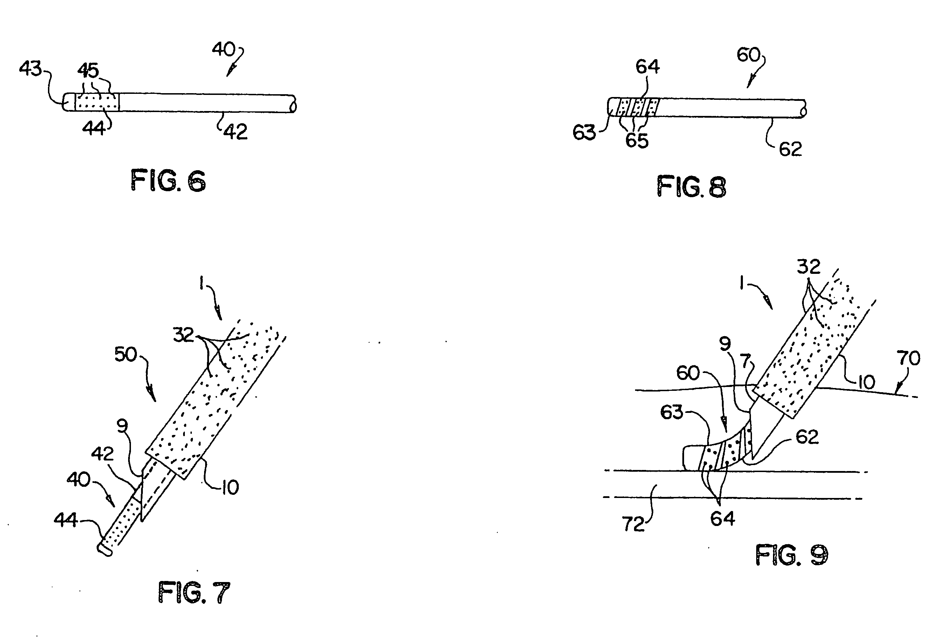

One example of a catheter having an echogenic capability is catheter 40, illustrated in Fig. 6. In the embodiment shown, catheter 40 includes a hollow catheter shaft 42 that is open at both ends and has a bore extending therethrough. Alternatively, the catheter may be closed at the distal end, and provided with one or more side ports for release of the fluid. Catheter 40 is preferably formed of a flexible polymeric material, and preferably includes a radiopaque filler. Non-limiting examples of suitable catheter materials include urethane, nylon, polyethylene, PTFE and silicone. The catheter outer diameter (O.D.) is typically dimensioned to be substantially equivalent to an 18, 19 or 20G cannula (1.27 mm [0.050 inch], 1.07 mm [0.042 inch], 0.89 mm [0.035 inch]). The catheter inner diameter (I.D.) is variable. It is desired to establish a balance in the catheter body between stiffness for insertion, and flow rate.

In the example shown in Fig. 6, the echogenic feature of the catheter is provided by fitting an echogenic band 44 on the distal end 43 of catheter shaft 42. Echogenic band 44 comprises a small cannula, preferably made of a metal or metal alloy. Examples of suitable cannula materials include stainless steel, nickel-titanium alloys such as nitinol, copper alloys and platinum. The cannula wall thickness is preferably about 0.10 mm [0.004 inch]. The cannula has a surface that is modified to incorporate deformations 45 along all or part of the surface of band 44. Deformations 45 may be formed in the same manner as deformations 32 formed along the needle. In order to apply band 44 to catheter shaft 42, the shaft may be stretched in longitudinal fashion to a smaller diameter, and the band is inserted thereover. When the stretching is relaxed, shaft 42 returns to its original diameter, and band 44 is snugly engaged therewith. Alternatively, a band may be placed within the lumen of the catheter, and the catheter may be tipped, locking the band in place. In this case, side ports can be provided for fluid flow. As still further alternatives, the band may be incorporated into the wall of the catheter, or the catheter matrix may be modified to include an echogenic material. Catheters having echogenic bands are known in the art for other purposes. Examples of such catheters include the ECHOTIP® ureteral catheter, and the ECHOTIP® Soft-Pass Embryo Transfer catheter, both available from Cook Incorporated of Bloomington, IN.

Fig. 7 illustrates a continuous nerve block assembly 50. Assembly 50 comprises echogenic needle 1, and includes catheter 40 passing through the hollow bore of the needle shaft and extending slightly (e.g., about 2-3 cm) beyond the distal end of the needle. Preferably, the catheter 40 includes a band 44 or like structure that provides an echogenic capability to the catheter. However, the assembly may alternatively utilize an echogenic needle in combination with a catheter that does not have an echogenic capability.

In Figs. 6 and 7, the echogenic surface of catheter 40 comprises band 44 applied thereto. Although band 44, and the associated deformations 45, are illustrated only at the distal end of catheter 40 shown in Figs. 6 and 7, the band can alternatively comprise a sheath having deformations 45 that extend all, or any designated part, of the length of catheter shaft 42. Alternatively, multiple bands can be spaced along the length of the catheter. Utilizing multiple bands, rather than a single elongated band, provides additional flexibility to the catheter that may not be available if an elongated metal or metal alloy sheath is utilized. As still further alternatives, the deformations applied to band 44 can have any size, shape and arrangement desired, including any of the arrangements of deformations 32 on the encasing sheath 30 for echogenic needle 1, as described above.

When needle 1 and catheter 40 are both provided with deformations, it is preferred that the deformations from each of the respective structures be sized, shaped, and/or arranged in a manner to provide a distinct visual image, or "footprint", for each. In this way, the ultrasound technician can readily distinguish the echogenic catheter from the echogenic needle (as the catheter exits the needle) under ultrasound.

When a catheter 40 extends distally beyond the bore of the needle 1 as illustrated in Fig. 7, the clinician, of course, must be careful not to puncture, or otherwise push against the nerve with the catheter in a manner that might cause damage to the nerve.

Fig. 8 illustrates a catheter 60 having an echogenic surface according to the present invention. Catheter 60 includes a hollow catheter shaft 62 that is open at both ends and has a bore extending therethrough, in the same manner as catheter 40. Alternatively, the distal end may be closed, and the shaft may be provided with one or more side ports for fluid flow. The invention refers to an echogenic coiled ribbon 64 is wrapped around all, or a portion, of the length of catheter shaft 62. Ribbon 64 may be formed of the same or a similar composition as band 44, such as a metal or metal alloy, and deformations 65 are disposed along the surface of the ribbon. Deformations 65 may be formed in the same manner as the deformations on the structures previously described, and may be dispersed along the surface of ribbon 64. Deformations 65 may be applied to ribbon 64 before coiling of the ribbon, or after coiling with the aid of a mandrel or other reinforcement. Echogenic ribbon 64 may be fitted over distal end 63 of shaft 62 by any convenient mechanism, such as wrapping it around the distal end, or by stretching the distal end of catheter shaft 62, and applying the ribbon to the stretched end, in the same manner as previously described for application of band 44 over shaft 42. As another alternative, ribbon 64 can be embedded into the wall of catheter 60, and a plastic can be extruded over the ribbon.

Utilizing a coiled ribbon 64 as described herein provides added flexibility to the distal end of the catheter when compared to the use of a solid echogenic band. If a needle is not substantially parallel with the nerve or nerves of interest within the neurovascular sheath during insertion of the catheter, the catheter may need to be turned as it exits the distal end of the needle, so as to minimize direct contact with the nerve. If a relatively inflexible band or other solid echogenic structure has been applied to the catheter, this structure limits the ability of the catheter to be able to turn or otherwise deflect until the band or other structure has passed through the needle. This can possibly cause damage to the nerve or other structures within the neurovascular sheath.

The added flexibility provided by the coiled ribbon enables the catheter tip to immediately turn or deflect as it exits the needle within the neurovascular sheath 70. This is illustrated in Fig. 9, wherein catheter 60 and ribbon 64 deflect when nerve 72 is encountered. The ability of the catheter distal end to deflect in this manner can avoid damage to the nerve that might otherwise be caused by excessive pressure or other blunt force caused by the catheter/cannula.

Those skilled in the art will appreciate that the use of a flexible catheter having an echogenic coiled ribbon as described hereinabove is not necessarily limited to use with an echogenic needle. Rather, the benefits achieved by virtue of visualizing the catheter with the coiled ribbon under ultrasound imaging may be extended to any use of echogenic catheters, such as the use with ureteral catheters and embryo transfer sets as described above.

As yet another alternative, instead of utilizing a separate echogenic band, ribbon, etc., the catheter may be formed to have an echogenic material incorporated into all or any designated portion of the catheter matrix. The incorporation of the echogenic material enables the technician to visualize the advancement of the catheter upon exiting the needle under ultrasound visualization.

It is therefore intended that the foregoing detailed description be regarded as illustrative rather than limiting, and that it be understood that it is the following claims, that are intended to define the scope of this invention.

## Claims

1. An echogenic catheter (40,60) suitable for ultrasonic visualization during a medical procedure, comprising:
a catheter shaft (42,62) having a proximal end, a distal end (43,63), and a bore extending at least partially therethrough;
wherein said catheter (40,60) is provided with a catheter echogenic surface disposed along at least a portion of a length of said catheter shaft (42,62) such that said catheter portion is visible under ultrasound visualisation **characterized in that** said catheter echogenic surface comprises a coiled ribbon (64), said coiled ribbon having deformations (65) formed thereon for enhancing reflection of ultrasound waves.

2. The echogenic catheter (40,60) of claim 1 wherein said catheter echogenic surface Is disposed along a distal portion of said catheter shaft (40,60).

3. The echogenlc catheter (40,60) of claim 2 wherein said distal portion of said catheter has a flexibility, and said colled ribbon (64) is disposed along said distal portion In a manner such that said flexibility Is substantially maintained.

4. The echogenlc catheter (40,60) of any preceding claim wherein said catheter shaft (42,62) is formed from a generally flexible polymeric composition.

5. The echogenic catheter (40,60) of claim 4, wherein said catheter shaft (42,62) Is formed from urethane, nylon, polyethylene, PTFE or silicone, and said coiled ribbon (64) comprises a metal of a metal alloy.

6. The echogenic catheter (40,60) of claim 5, wherein said catheter shaft (42,62) Is formed from urethane or nylon, and said colled ribbon (64) comprises stainless steel, a nickel-titanium alloy, a copper alloy or platinum.

7. The echogenic catheter (40,60) of any preceding claim wherein the colled ribbon (64) is fitted over the distal end of said catheter shaft (43,63),

8. The echogenlc catheter (40,60) of any preceding claim wherein the coiled ribbon (64) is wrapped around at least a distal portion of said catheter (40,60),

9. The echogenic catheter (40,60) of any preceding claim wherein the coiled ribbon (64) is embedded into at least a distal portion of said catheter (40,60),

10. The echogenic catheter (40,60) of claim 9 wherein a plastic is extruded over the coiled ribbon (64).

11. An assembly for providing continuous blocking of a nerve of a patient, comprising:
a needle (1) comprising a hollow needle conduit (2) having a shaft portion (30) and a tip portion (9), a generally non-conductive layer (7) disposed along a length of said conduit shaft portion (30), and an echogenic surface (10) extending along at least a portion of said needle; and
a catheter (40,60) according to any preceding claim ; said catheter (40,60) sized to be received In said shaft portion (30) of said needle (1), and said catheter shaft (42,62) having a bore for transmittal of anaesthetic to a vicinity of said nerve.

12. The assembly of claim 11, wherein said needle (1) is capable of providing electrical stimulation to said nerve, and said respective needle and catheter echogenic surfaces (10,44,64) are arranged for enhancing a reflection of ultrasound waves generated during said ultrasound visualization.

13. The assembly of any of claims 11 and 12, wherein said respective echogenic surfaces (10,44,64) are arranged such that a reflection of ultrasound waves from said needle echogenic surface (10) generated during ultrasound visualization is visually distinct from a reflection of ultrasound waves from said catheter echogenic surface (44,64).

## Patentansprüche

1. Echogener Katheter (40, 60), der für die Ultraschallvisualisierung während eines medizinischen Verfahrens geeignet ist, der Folgendes umfasst:
einen Katheterschaft (42, 62) mit einem proximalen Ende, einem distalen Ende (43, 63) und einer Bohrung, die sich mindestens teilweise dort hindurch erstreckt;
wobei der Katheter (40, 60) mit einer echogenen Katheteroberfläche, die mindestens entlang eines Abschnittes einer Länge des Katheterschaftes (42, 62) angeordnet ist, derart versehen ist, dass der Katheterabschnitt unter Ultraschallvisualisierung sichtbar ist, **dadurch gekennzeichnet, dass** die echogene Oberfläche ein gewickeltes Band (64) aufweist; wobei das gewickelte Band Formveränderungen (65) aufweist, die zur Erhöhung der Reflexion von Ultraschallwellen darauf ausgebildet sind.

2. Echogener Katheter (40, 60) nach Anspruch 1, wobei die echogene Katheteroberfläche entlang einem distalen Abschnitt von dem Katheterschaft (40, 60) angeordnet ist.

3. Echogener Katheter (40, 60) nach Anspruch 2, wobei der distale Abschnitt des Katheters eine Flexibilität aufweist und das gewickelte Band (64) entlang dem distalen Abschnitt derart angeordnet ist, dass die Flexibilität im Wesentlichen erhalten bleibt.

4. Echogener Katheter (40, 60) nach einem der vorangehenden Ansprüche, wobei der Katheterschaft (42, 62) aus einer üblicherweise elastischen Polymerzusammensetzung gebildet ist.

5. Echogener Katheter (40, 60) nach Anspruch 4, wobei der Katheterschaft (42, 62) aus Urethan, Nylon, Polyethylen, PTFE oder Silikon gebildet ist und das gewickelte Band (64) ein Metall oder eine Metalllegierung umfasst.

6. Echogener Katheter (40, 60) nach Anspruch 5, wobei der Katheterschaft (42, 62) aus Urethan oder Nylon gebildet ist und das gewickelte Band (64) Edelstahl, eine Nickel-Titan-Legierung, eine Kupferlegierung oder Platin umfasst.

7. Echogener Katheter (40, 60) nach einem der vorangehenden Ansprüche, wobei das gewickelte Band (64) über dem distalen Ende von dem Katheterschaft (43, 63) montiert ist.

8. Echogener Katheter (40, 60) nach einem der vorangehenden Ansprüche, wobei das gewickelte Band (64) mindestens um einen distalen Abschnitt von dem Katheter (40, 60) herumgewickelt ist.

9. Echogener Katheter (40, 60) nach einem der vorangehenden Ansprüche, wobei das gewickelte Band (64) mindestens in einen distalen Abschnitt von dem Katheter (40, 60) eingelassen ist.

10. Echogener Katheter (40, 60) nach Anspruch 9, wobei ein Kunststoff über das gewickelte Band (64) extrudiert ist.

11. Anordnung zum Bereitstellen der kontinuierlichen Blockierung eines Nervs von einem Patienten, die Folgendes umfasst:
eine Nadel (1), die eine durchgehende Hohlnadel (2) mit einem Schaftabschnitt (30) und einem Spitzenabschnitt (9) umfasst, einer gewöhnlich nicht-leitenden Schicht (7), die entlang einer Länge des durchgehenden Schaftabschnittes (30) angeordnet ist, und eine echogene Fläche (10), die sich mindestens entlang eines Abschnittes der Nadel erstreckt; und
einen Katheter (40, 60) nach einem der vorangehenden Ansprüche, wobei der Katheter (40, 60) so bemessen ist, dass er in den Schaftabschnitt (30) der Nadel (1) aufgenommen wird und der Katheterschaft (42, 62) eine Bohrung für die Übertragung eines Narkosemittels in die Nähe von dem Nerv aufweist.

12. Anordnung nach Anspruch 11, wobei die Nadel (1) in der Lage ist, eine elektrische Stimulation des Nervs bereitzustellen und die entsprechenden Nadel- und echogenen Katheterflächen (10, 44, 64) zur Erhöhung einer Reflexion der Ultraschallwellen, die während der Ultraschallvisualisierung erzeugt werden, ausgeführt sind.

13. Anordnung nach einem der Ansprüche 11 und 12, wobei die entsprechenden echogenen Flächen (10, 44, 64) derart ausgeführt sind, dass eine Reflexion der Ultraschallwellen, die während der Ultraschallvisualisierung erzeugt werden, von der echogenen Nadelfläche (10) sich optisch von einer Reflexion der Ultraschallwellen von der echogenen Katheterfläche (44, 64) unterscheidet.

## Revendications

1. Cathéter échogénique (40, 60) permettant la visualisation par ultrasons pendant une opération médicale, le cathéter comprenant :
une tige de cathéter (42, 62) qui présente une extrémité proximale et une extrémité distale (43, 63) et qui est traversée au moins partiellement par un alésage,
ledit cathéter (40, 60) étant doté d'une surface échogénique de cathéter placée le long d'au moins une partie de la longueur de ladite tige de cathéter (42, 62) de manière à ce que ladite partie de cathéter soit visible par visualisation par ultrasons, ladite surface échogénique de cathéter présentant un ruban enroulé (64), ledit ruban enroulé présentant sur sa surface des déformations (65) améliorant la réflexion des ondes ultrasoniques.

2. Cathéter échogénique (40, 60) selon la revendication 1, dans lequel ladite surface échogénique est placée le long d'une partie distale de ladite tige de cathéter (40, 60).

3. Cathéter échogénique (40, 60) selon la revendication 2, dans lequel ladite partie distale dudit cathéter est flexible et ledit ruban enroulé (64) est placé le long de ladite partie distale de manière à maintenir essentiellement ladite flexibilité.

4. Cathéter échogénique (40, 60) selon une quelconque des revendications précédentes, dans lequel ladite tige de cathéter (42, 62) est formée d'une composition polymère globalement flexible.

5. Cathéter échogénique (40, 60) selon la revendication 4, dans lequel ladite tige de cathéter (42, 62) est formée d'uréthane, de nylon, de polyéthylène, de PTFE ou de silicone, ledit ruban enroulé (64) contenant un métal ou un alliage métallique.

6. Cathéter échogénique (40, 60) selon la revendication 5, dans lequel ladite tige de cathéter (42, 62) est formée d'uréthane ou de nylon et ledit ruban enroulé (64) contient de l'acier inoxydable, un alliage de nickel et de titane, un alliage de cuivre ou du platine.

7. Cathéter échogénique (40, 60) selon l'une quelconque des revendications précédentes, dans lequel le ruban enroulé (64) est placé sur l'extrémité distale de ladite tige de cathéter (43, 63).

8. Cathéter échogénique (40, 60) selon l'une quelconque des revendications précédentes, dans lequel le ruban enroulé (64) est enroulé autour d'au moins la partie distale dudit cathéter (40, 60).

9. Cathéter échogénique (40, 60) selon l'une quelconque des revendications précédentes, dans lequel ledit ruban enroulé (64) est incorporé dans au moins une partie distale dudit cathéter (40, 60).

10. Cathéter échogénique (40, 60) selon la revendication 9, dans lequel une matière plastique est extrudée au-dessus du ruban enroulé (64).

11. Ensemble permettant le blocage permanent d'un nerf d'un patient, comprenant :
une aiguille (1) comprenant un conduit creux d'aiguille (2) qui présente une partie de tige (30) et une partie de pointe (9), une couche (7) globalement non conductrice placée le long d'une longueur de ladite partie de tige (30) de conduit et une surface échogénique (10) qui s'étend sur au moins une partie de ladite aiguille et
un cathéter (40, 60) selon l'une quelconque des revendications précédentes, ledit cathéter (40, 60) étant dimensionné pour être reçu dans ladite partie de tige (30) de ladite aiguille (1) et ladite tige de cathéter (42, 62) présentant un alésage qui permet de transmettre un anesthésique local au voisinage dudit nerf.

12. Ensemble selon la revendication 11, dans lequel ladite aiguille (1) permet de délivrer une stimulation électrique audit nerf, ladite aiguille et lesdites surfaces échogéniques (10, 44, 64) de cathéter respectives étant agencées de manière à améliorer la réflexion des ondes ultrasoniques produites pendant ladite visualisation par ultrasons.

13. Ensemble selon l'une quelconque des revendications 11 à 12, dans lequel lesdites surfaces échogéniques (10, 44, 64) respectives sont agencées de telle sorte de la réflexion des ondes ultrasoniques produites par ladite surface échogénique (10) d'aiguille pendant la visualisation par ultrasons soit visuellement distincte de la réflexion d'ondes ultrasoniques émises par ladite surface échogénique (44, 64) de cathéter.
